# EUROPEAN PATENT APPLICATION

(11) **EP 4 424 845 A1**
(43) Date of publication of application: **04.09.2024**
(21) Application number: 24160781.1
(22) Date of filing: 01.03.2024
(51) Int. Cl.: C12Q 1/6874, C12Q 1/6869

(54) **METHOD, KIT AND CARTRIDGE FOR DETECTING NUCLEIC ACID MOLECULE**

(30) Priority: 01.03.2023 US 202363449284 P
(71) Applicant: Personal Genomics Taiwan, Inc., 30261 Hsinchu County (TW)
(72) Inventor: CHIOU, Chung-Fan, 307 Hsinchu County (TW); CHEN, Bor-Huah, 402 Taichung City (TW)
(74) Representative: Becker, Eberhard

(57) **Abstract**

A method for generating high accuracy sequencing results from low accuracy continuous single molecule sequencing includes the following steps: providing a sample including first target nucleic acid molecules; hybridizing first capture primers to the first target nucleic acid molecules to form first capture primer-first target nucleic acid molecule complexes; performing a nascent strand synthesis reaction to form first template molecule-first target nucleic acid molecule complexes; dissociating first template molecules; hybridizing first sequencing primers to the dissociated first template molecules, and sequencing the first template molecules by single-molecule sequencing to obtain first original sequences; comparing the first original sequences and a first target sequence to calculate a first sequence similarity of each of the first original sequences; collecting first original sequences whose first sequence similarity reaches a predetermined threshold to obtain a first cluster; and analyzing the first cluster by bioinformatics to obtain a first consensus sequence.

## Description

### BACKGROUND

### Technical Field

The present invention relates to a detection method, a detection kit and a detection cartridge, and in particular to a method, a kit and a cartridge for detecting nucleic acid molecule.

### Description of Related Art

Continuous single molecule sequencing technologies are capable for directly reading sequences from native nucleic acid molecules without the need for nucleic acid amplification steps. Despite the remarkable sequencing speed of single molecule sequencing, reaching up to 5 bases per second, significantly faster than Next Generation Sequencing (NGS) which typically takes over 2 minutes to read a single base, the raw read accuracy remains a challenge, barely reaching 85%. This level of accuracy falls short for many applications, particularly in medical diagnostics and healthcare.
Efforts to improve sequencing accuracy involve developments in both upstream and downstream procedures. One well-known approach is the construction of circular templates for sequencing, enabling repeated reading of templates and alignment of raw reads to generate more accurate consensus results. While this circular template method effectively enhances sequencing accuracy to meet application requirements, its complex manipulation procedures and lengthy operation time limit its applicability. There is a pressing need to develop a simple and rapid protocol to meet the accuracy requirements of single molecule sequencing and broaden its application scope.

### SUMMARY

The present invention provides a method, a kit and a cartridge for detecting nucleic acid molecule, which may simplify detection steps, reduce detection time and improve detection accuracy as well as provide rich information.

The present invention provides a method for detecting nucleic acid molecule. The method includes the following steps: providing a sample including first target nucleic acid molecules; hybridizing first capture primers to the first target nucleic acid molecules to form first capture primer-first target nucleic acid molecule complexes; performing a nascent strand synthesis reaction to form first template molecule-first target nucleic acid molecule complexes; dissociating first template molecules; hybridizing first sequencing primers to the dissociated first template molecules, and sequencing the first template molecules by single-molecule sequencing to obtain first original sequences (raw read sequences); comparing the first original sequences with a first target sequence to calculate a first sequence similarity of each of the first original sequences; collecting first original sequences whose first sequence similarity reaches a predetermined threshold to obtain a first cluster; and analyzing the first cluster by bioinformatics to obtain a first consensus sequence. The plurality of first capture primers have linkers, and the linkers are paired with another linker on a sequencing chip.

In one embodiment of the present invention, the sample is unpurified or crudely purified, which further includes a plurality of non-target nucleic acid molecules, and the plurality of non-target nucleic acid molecules do not hybridize by the plurality of first capture primers or the plurality of first sequencing primers.

In one embodiment of the present invention, the plurality of first target nucleic acid molecules are not amplified by polymerase chain reaction.

In one embodiment of the present invention, the plurality of first target nucleic acid molecules include DNA, mRNA, miRNA or miniRNA.

In one embodiment of the present invention, pairing method includes biotin-streptavidin binding, nucleic acid hybridization or chemical bonding.

In one embodiment of the present invention, the plurality of first template molecules are single-stranded and linear nucleic acids.

In one embodiment of the present invention, the plurality of first template molecules have 25 to 10,000 nucleotides.

In one embodiment of the present invention, the method further includes: after forming the plurality of first template molecule-first target nucleic acid molecule complexes, performing a cleaning process and a purification process.

In one embodiment of the present invention, the method further includes: after dissociating the plurality of first template molecules and before hybridizing the plurality of first sequencing primers to the plurality of dissociated first template molecules, immobilizing the plurality of dissociated first template molecules on a sequencing chip.

In one embodiment of the present invention, the method further includes: before hybridizing the plurality of first capture primers to the plurality of first target nucleic acid molecules, immobilizing the plurality of first capture primers on a sequencing chip.

In one embodiment of the present invention, the plurality of first template molecules includes a first end and a second end, the first end includes the first capture primer, and the second end is hybridized by the plurality of first sequencing primers.

In one embodiment of the present invention, the method further includes: calculating an error mode distribution of each nucleotide in the first consensus sequence to determine whether the plurality of first target nucleic acid molecules have variation.

In one embodiment of the present invention, the variation includes nucleotide insertion, nucleotide deletion, nucleotide substitution, gene recombination and/or methylation site.

In one embodiment of the present invention, the predetermined threshold is greater than 80%.

In one embodiment of the present invention, the sample further includes a plurality of second target nucleic acid molecules, and the method further includes the following steps: hybridizing a plurality of second capture primers to the plurality of second target nucleic acid molecules to form a plurality of second capture primer-second target nucleic acid molecule complexes; performing a nascent strand synthesis reaction to form a plurality of second template molecule-second target nucleic acid molecule complexes; dissociating a plurality of second template molecules; hybridizing a plurality of second sequencing primers to the plurality of dissociated second template molecules, and sequencing the plurality of second template molecules by single-molecule sequencing to obtain a plurality of second original sequences; comparing the plurality of second original sequences and a second target sequence to calculate a second sequence similarity of each of the plurality of second original sequences; collecting a plurality of second original sequences whose second sequence similarity reaches a predetermined threshold to obtain a second cluster; and analyzing the second cluster by bioinformatics to obtain a second consensus sequence.

In one embodiment of the present invention, the method further includes: simultaneously detecting the plurality of first target nucleic acid molecules and the plurality of second target nucleic acid molecules.

In one embodiment of the present invention, the number of the plurality of first original sequences in the cluster is counted to determine copy number and expression level of the plurality of first target nucleic acid molecules.

The present invention provides a kit for detecting nucleic acid molecule. The kit uses the aforementioned method to detect a plurality of first target nucleic acid molecule and a plurality of second target nucleic acid molecules in a sample simultaneously. The kit includes a mix of a plurality of first capture primers and a plurality of second capture primers, a mix of a plurality of first sequencing primers and a plurality of second sequencing primers, a first reagent for performing a nascent strand synthesis reaction, a sequencing chip, and a second reagent for single-molecule sequencing.

In above mentioned kit, the mix of capture primers and the mix of sequencing primer can be provided by the user.

In one embodiment of the present invention, the capture primers and the sequencing primers are not provided in advance; on the contrary, after the plurality of first target nucleic acid molecule to be detected is confirmed, a plurality of required capture primers and a plurality of required sequencing primers are added to the kit.

The present invention provides a cartridge for detecting nucleic acid molecule. The cartridge uses the aforementioned method to detect a plurality of first target nucleic acid molecules and a plurality of second target nucleic acid molecules within a sample simultaneously. The cartridge comprises a first chamber for adding the sample during detection, a second chamber housing a mix of a plurality of first capture primers and a plurality of second capture primers and a sequencing chip, a third chamber containing a mix of a plurality of first sequencing primers and a plurality of second sequencing primers, a fourth chamber containing a first reagent for performing nascent strand synthesis reaction, and a fifth chamber containing a second reagent for single-molecule sequencing. The plurality of first capture primers and the plurality of second capture primers are immobilized on the sequencing chip.

In the aforementioned cartridge, the mix of capture primers and the mix of sequencing primer can be provided by the user.

In one embodiment of the present invention, the capture primers and the sequencing primers are not provided in advance; on the contrary, after the plurality of first target nucleic acid molecule to be detected is confirmed, a plurality of required capture primers and a plurality of required sequencing primers are added to the chambers of cartridge.

Based on the above, in the method, the kit and the cartridge for detecting nucleic acid molecule according to an embodiment of the present invention, the detection steps are simplified, the detection time is reduced and the detection accuracy is improved by using a specific sequence of steps. The specific sequence of steps at least includes the following steps: first hybridizing the capture primers to the target nucleic acid molecules in the unpurified or crudely purified sample, then obtaining the template molecules by the nascent strand synthesis reaction, then hybridizing the sequencing primers to the template molecules, then obtaining original sequences of the template molecules by the single-molecule sequencing, and then analyzing the original sequences whose sequence similarity reaches a predetermined threshold to obtain the consensus sequence. In this embodiment, the specific sequence of steps combines a simple preparation method and bioinformatics. The simple preparation method reads targeted nucleic acid fragments during the single-molecule sequencing and reads the nucleic acid fragments across multiple genomes in the sample. The bioinformatics assembles the low-accuracy original sequences obtained from each individually read nucleic acid fragment into a high-accuracy result that can meet application requirements.

To make the aforementioned more comprehensible, several embodiments accompanied with drawings are described in detail as follows.

### BRIEF DESCRIPTION OF THE DRAWINGS

The accompanying drawings are included to provide a further understanding of the disclosure, and are incorporated in and constitute a part of this specification. The drawings illustrate exemplary embodiments of the disclosure and, together with the description, serve to explain the principles of the disclosure.
FIG. 1 is a flow chart of a method for detecting nucleic acid molecules according to an embodiment of the present invention.
FIG. 2A to FIG. 2I are schematic diagrams of a method for detecting nucleic acid molecules according to an embodiment of the present invention.
FIG. 3 is a schematic diagram of a method for detecting nucleic acid molecules according to another embodiment of the present invention.
FIG. 4A shows the result of using original sequences whose sequence similarity reaches the predetermined threshold to generate the consensus sequence.
FIG. 4B shows the result of comparing the consensus sequence with the reference sequence.

### DESCRIPTION OF THE EMBODIMENTS

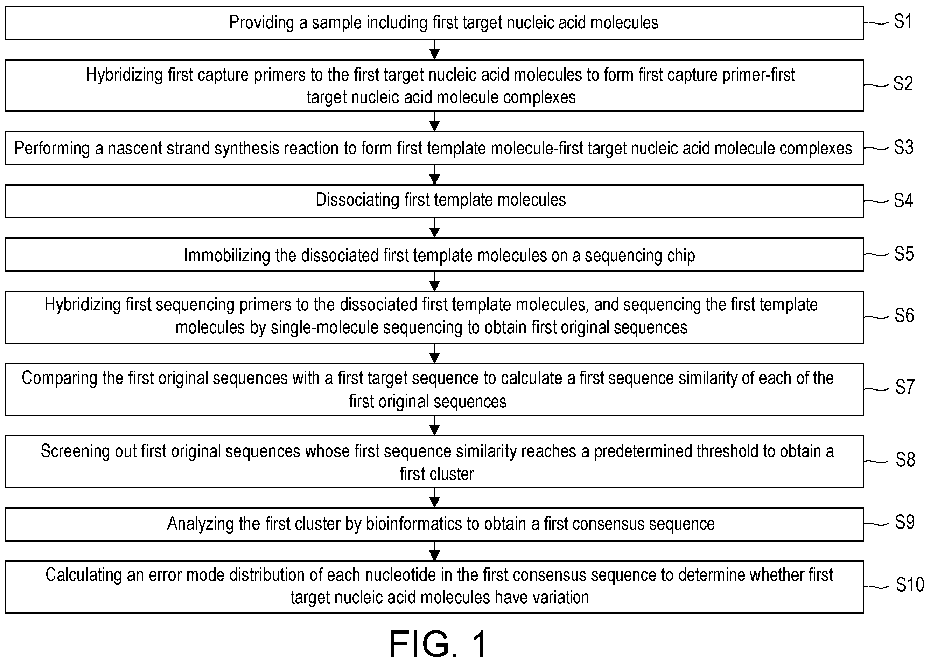

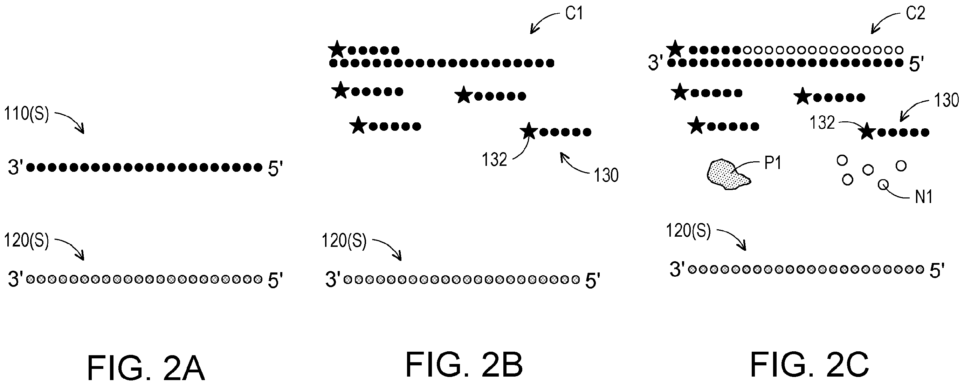

FIG. 1 is a flow chart of a method for detecting nucleic acid molecules according to an embodiment of the present invention. FIG. 2A to FIG. 2I are schematic diagrams of a method for detecting nucleic acid molecules according to an embodiment of the present invention. The method for detecting nucleic acid molecules in this embodiment may include the following steps:

First, referring to FIG. 1 and FIG. 2A at the same time, in step S 1, a sample S is provided. In this embodiment, the sample S may be derived from biological materials such as blood, cell lines, tissues, or microorganisms (such as viruses and bacteria), but the invention is not limited thereto. The sample S may be an unpurified extract that extracted from the biological material, a roughly purified extract that extracted and roughly purified from the biological material, or purified nucleic acid molecules that extracted and purified from the biological material, but the invention is not limited thereto.

In this embodiment, the sample S may include a plurality of first target nucleic acid molecules 110 and a plurality of non-target nucleic acid molecules 120. The first target nucleic acid molecule 110 may be defined as a nucleic acid molecule that may be hybridized by a first capture primer and a first sequencing primer used in the subsequent detection step and may have high sequence similarity with a first target sequence. The non-target nucleic acid molecule 120 may be defined as a nucleic acid molecule that may not be hybridized by the first capture primer and the first sequencing primer used in the subsequent detection step and may have low sequence similarity with the first target sequence. The first target sequence may be a known sequence, and the first target sequence may be, for example, a reference sequence in a database (such GenBank or Genome Database), but the invention is not limited thereto. In this embodiment, the first target nucleic acid molecule 110 may be DNA, and the non-target nucleic acid molecule 120 may also be DNA, but the invention is not limited thereto. In some embodiments, the first target nucleic acid molecule may also be various types of RNA (such as mRNA, miRNA or miniRNA) or other types of DNA (such as DNA with methylation modification). In some embodiments, non-target nucleic acid molecules may also be various types of RNA (such as mRNA, miRNA, or miniRNA) or other forms of DNA (such as DNA with methylation modifications).

Compared to general detection methods that use nucleic acid molecules amplified by polymerase chain reaction (PCR) for detection, the first target nucleic acid molecule 110 used in this embodiment is nucleic acid molecule that is original and has not been amplified by polymerase chain reaction, thereby preventing sequence of the detected first target nucleic acid molecule from being distorted due to amplification. For example, sequence of nucleic acid molecules amplified by polymerase chain reaction usually has a certain probability of errors (such as mutations), and the errors (mutations) may also be amplified through continued polymerase chain reactions, resulting in distortion of the sequence of the amplified nucleic acid molecules. In addition, when performing polymerase chain reaction, non-target nucleic acid molecules may be mistakenly amplified due to poor specificity of primer, resulting in distortion of the sequence of the amplified nucleic acid molecules.

Then, referring to FIG. 1 and FIG. 2B at the same time, in step S2, the first capture primer 130 is hybridized to the first target nucleic acid molecule 110 to form a first capture primer-first target nucleic acid molecule complex C1. In this embodiment, since the first capture primer 130 has a nucleic acid sequence complement with the first target nucleic acid molecule 110, the first capture primer 130 may anneal to the first target nucleic acid molecule 110 to form the first capture primer-first target nucleic acid molecule complex C1. The first capture primer 130 may be, for example, an oligonucleotide, but the invention is not limited thereto.

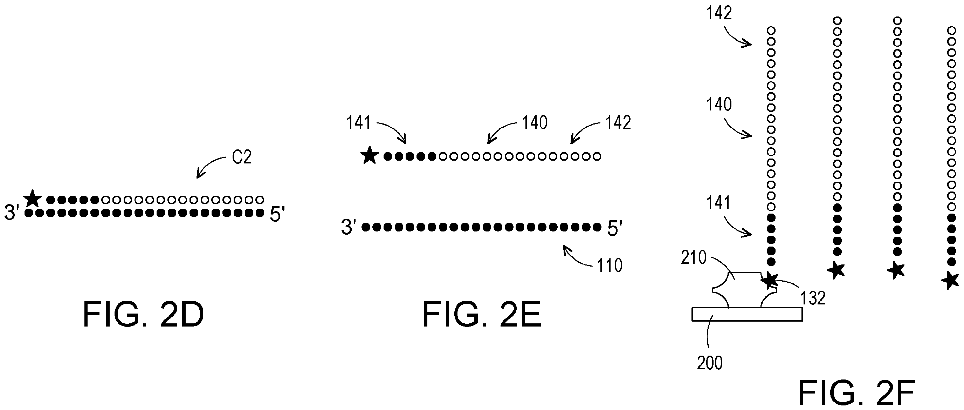

In this embodiment, the first capture primer 130 has a linker 132. The linker 132 may be used to pair with another linker 210 of a sequencing chip 200 in subsequent steps, thereby allowing the first capture primer 130 be immobilized on the sequencing chip 200 by pairing the linker 132 with the other linker 210, as shown in FIG. 2F.

Then, referring to FIG. 1 and FIG. 2C at the same time, in step S3, a nascent strand synthesis reaction is performed to form a first template molecule-first target nucleic acid molecule complex C2. Specifically, after the first capture primer-first target nucleic acid molecule complex C1 is formed, the nucleic acid sequence of the first target nucleic acid molecule 110 is used as a template, and a polymerase P1 is used to sequentially join appropriate deoxyribonucleoside triphosphates (dNTP) N1 to an 3' end of the first capture primer 130 in the first capture primer-first target nucleic acid molecule complex C1 to form the first template molecule-first target nucleic acid molecule complex C2 containing a nascent strand (that is, the first template molecule 140).

In some embodiments, when the first target nucleic acid molecule is various types of RNA (such as mRNA, miRNA or miniRNA), in step S3, a reverse transcriptase is used to replace a polymerase P1 to perform a nascent strand synthesis reaction. The reverse transcriptase may be, for example, M-MLV RTase, but the invention is not limited thereto.

Then, referring to FIG. 1 and FIG. 2D at the same time, after forming the first template molecule-first target nucleic acid molecule complex C2, a cleaning process and a purification process are performed. The cleaning process may be used to remove impurities (such as non-target nucleic acid molecules 120, unreacted first capture primer 130, a polymerase P1, unreacted deoxyribonucleoside triphosphate N1, etc.), and the purification process may be used to increase concentration of the first template molecule 140, thereby reducing the interference caused by impurities, improving the efficiency of subsequent immobilizing the first template molecule on the sequencing chip, and improving the efficiency of sequencing.

Then, referring to FIG. 1 and FIG. 2E at the same time, in step S4, the first template molecule 140 is dissociated. Specifically, after the first template molecule-first target nucleic acid molecule complex C2 is formed, denaturing condition such as heating is used to dissociate the first template molecule 140 in the first template molecule-first target nucleic acid molecule complex C2 and expose the linker 132 in the dissociated first template molecule 140. In this embodiment, the first template molecule 140 may be a single-stranded and linear nucleic acid. The first template molecule 140 has, for example, 25 to 10,000 nucleotides, but the invention is not limited thereto. The first template molecule 140 includes a first end 141 and a second end 142 opposite to each other. The first end 141 includes the first capture primer 130, and the second end 142 is used to be hybridized by the first sequencing primer 150 in the subsequent step.

Compared to general detection methods that require complicated steps to prepare a circular template (such as a circular nucleic acid) for sequencing, the first template molecule 140 used for sequencing in this embodiment is single-stranded and linear nucleic acid, thereby simplifying the detection steps and reducing detection time.

Compared to general detection methods that use template molecule with short fragments (for example, less than 1000 nucleotides) for sequencing and require providing more sequencing results to recombine the target sequence, the first template molecule 140 used for sequencing in this embodiment is a long fragment of up to 10,000 nucleotides, whereby the target sequence may be recombined without providing sequencing results that are dozens of times the size of the target sequence, thereby simplifying the detection steps and reducing detection time.

Then, referring to FIG. 1 and FIG. 2F at the same time, in step S5, the dissociated first template molecule 140 is immobilized on the sequencing chip 200. Specifically, the first template molecule 140 is immobilized on the sequencing chip 200 by pairing the linker 132 of the first template molecule 140 with the other linker 210 of the sequencing chip 200. In this embodiment, the linker 132 may be, for example, biotin, and the other linker 210 may be, for example, streptavidin; and the linker 132 may be paired with the other linker 210 are by biotin-streptavidin binding, but the invention is not limited thereto. In some embodiments, linker and other linker are not biotin and streptavidin respectively, and the pairing method may be nucleic acid hybridization or chemical bonding.

In this embodiment, the first template molecule 140 containing the first capture primer 130 is immobilized on the sequencing chip 200 after the first template molecule 140 is dissociated and before the first sequencing primer 150 is hybridized to the dissociated first template molecule 140, but the invention is not limited thereto. In some embodiments, the first capture primer may also be immobilized on the sequencing chip before the first capture primer is hybridized to the first target nucleic acid molecule.

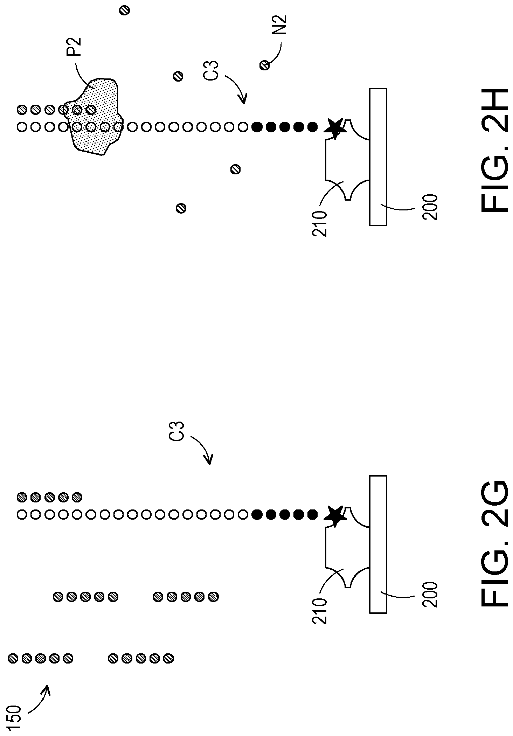

Then, referring to FIG. 1 and FIG. 2G at the same time, in step S6, the first sequencing primer 150 is hybridized to the dissociated first template molecule 140. In this embodiment, after the dissociated first template molecule 140 is immobilized on the sequencing chip 200, another cleaning process may be optionally performed to remove first template molecules 140 that are not immobilized on the sequencing chip 200, and then adding the first sequencing primer 150. In this embodiment, since the first sequencing primer 150 has a nucleic acid sequence complement with the first template molecule 140, the first sequencing primer 150 may anneal to the first template molecule 140 to form a first sequencing primer-first template molecular complex C3. The first sequencing primer 150 may be, for example, an oligonucleotide, but the invention is not limited thereto.

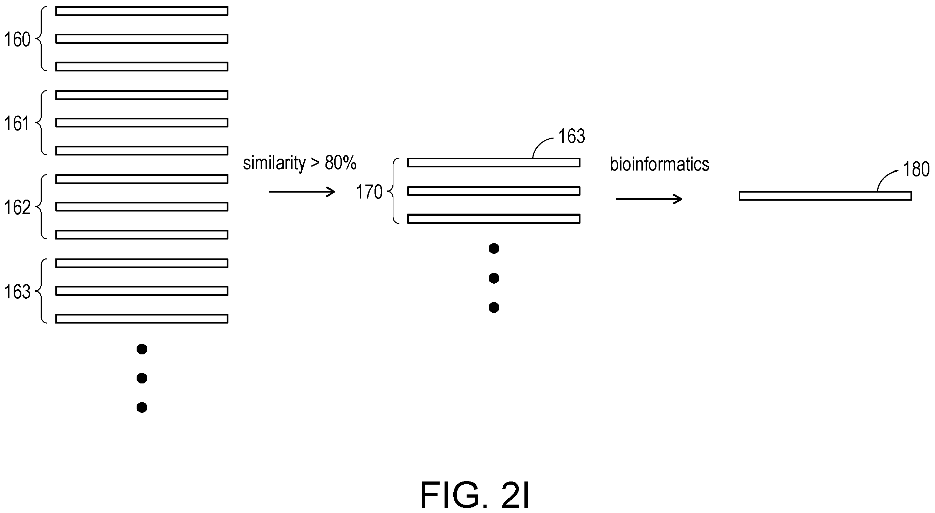

Then, referring to FIG. 1 and FIG. 2H at the same time, in step S6, sequencing the first template molecule 140 by single-molecule sequencing to obtain first original sequence. Specifically, after the first sequencing primer-first template molecule complex C3 is formed, the nucleic acid sequence of the first template molecule 140 is used as a template, and a polymerase P2 is used to sequentially join appropriate fluorescence-labelled nucleotide analogs N2 to an 3' end of the first sequencing primer 150 in the first sequencing primer-first template molecule complex C3 to sequence the nucleic acid sequence of the first template molecule 140 and obtain first original sequences 160, 161, 162, 163, as shown in FIG. 2I.

Then, referring to FIG. 1 and FIG. 2I at the same time, in step S7, the first original sequences 160, 161, 162, 163 are compared with a first target sequence to calculate a first sequence similarity of the first original sequences 160, 161, 162, 163. For example, the first sequence similarity of the first original sequences 160 is about 65%, the first sequence similarity of the first original sequences 161 is about 70%, the first sequence similarity of the first original sequences 162 is about 79%, and the first sequence similarity of the first original sequences 163 is about 88%. Each original sequence in the first original sequences 160 (or the first original sequences 161, or the first original sequences 162, or the first original sequences 163) may be the same or different.

Then, referring to FIG. 1 and FIG. 2I at the same time, in step S8, the first original sequences 160, 161, and 162 whose first sequence similarity does not reach a predetermined threshold are excluded, and the first original sequences 163 whose first sequence similarity reaches a predetermined threshold are collected to obtain a first cluster 170. In this embodiment, the predetermined threshold is, for example, greater than 80% to improve the accuracy of the first consensus sequence 180 obtained in subsequent steps, but the invention is not limited thereto. In some embodiments, the predetermined threshold may also be greater than 80%, 85% or 95%, to further improve the accuracy of the first consensus sequence 180 obtained in subsequent steps. In some embodiments, the predetermined threshold may be determined based on error rate of the used single-molecule sequencing method. For example, when the error rate of the single-molecule sequencing method is 15% (i.e., accuracy 85%), the predetermined threshold may be 80% (85% -5%=80%).

Then, referring to FIG. 1 and FIG. 2I at the same time, in step S9, the first cluster 170 is analyzed by bioinformatics to obtain a first consensus sequence 180 with high accuracy.

Compared to general detection methods that only use probe hybridization to identify nucleic acid molecules and are prone to misjudgment, this embodiment uses the single-molecule sequencing and the bioinformatics to ensure that the first original sequence of the collected first template molecule 140 is derived from the first target nucleic acid molecule, thereby reducing the probability of misjudgment and improving the detection accuracy. For example, when a non-target nucleic acid molecule is mistakenly captured by the first capture primer 130, mistakenly hybridized by the first sequencing primer 150 and sequenced to obtain an original sequence, using the single-molecule sequencing and the bioinformatics may clearly known that the original sequence is derived from the non-target nucleic acid molecule rather than from a target nucleic acid molecule (that is, sequence similarity of the original sequence derived from the non-target nucleic acid molecule should be less than 80%), thereby excluding the original sequence derived from the non-target nucleic acid molecule.

Then, referring to FIG. 1, in step S10, an error mode distribution of each nucleotide in the first consensus sequence 180 is calculated to determine whether the first target nucleic acid molecule has a variation or mutation. Specifically, aligning and checking whether each nucleotide in the first consensus sequence 180 is the same as or different from each corresponding nucleotide in each first original sequence 163 of the first cluster 170, and then calculating an error mode distribution of each nucleotide in the first consensus sequence 180.

When encountering a targeted nucleic acid fragment in the sequencing sample with multiple types of mutations, we are able to detect and determine the specific mutation types, as well as their frequencies, by generating consensus sequences and analyzing the sequence information therein.

Single molecule sequencing technologies produce random errors with a predetermined probability. The error probabilities are systemically correlated, with each sequencing platform generating a consistent error probability profile.

Below, we offer a comprehensive example illustrating the detection of polymorphisms and the estimation of their respective ratios.

Consider a scenario involving a single molecule sequencing platform with an accuracy rate of 90%, corresponding to a 10% error rate per base read event. Error modes and their probabilities are categorized as following: N (accurate reading) at 90%, E1, E2, E3 (misreading the base as one of the other three bases), each at 2%, and O (skipping the base, resulting in no reading), at 4%. These percentages denote the likelihood of each error mode occurring.

Let's assume the collection of 1000 original sequencing reads within one cluster. For a sample devoid of any mutation types, suppose the base at position 102 of the molecule being read is "A". Consequently, among the 1000 original reads in the cluster, the outcome at position 102 will approximately comprise "A" around 900 times, "T" 20 times, "C" 20 times, "G" 20 times and "O" (skipped base) 40 times, resulting from the calculation "A" 1000 x 90% = 900, and similarly for other bases). Hence, the read result (error mode distribution) at position 102 will roughly manifest as (A 900, T 20, C 20, G 20, O 40).

Now, let's assume a sample exhibiting a single nucleotide polymorphism at position 102, with the "A" type comprising 75% and the "T" type comprising 25%. Given that the capture primer hybridizes to the sample fragment randomly, with an equal probability for each polymorphisms, the resulting original reads within the cluster will reflect the 75% to 25% ratio of "A" and "T" polymorphisms.

Consequently, among the 1000 original reads in the cluster, approximately 750 originate from the "A" polymorphism, while around 250 result from the presence of the "T" polymorphism. The 750 "A" type will yield a result of approximately (A 675, T 15, C 15, G 15, O 30), and the 250 "T" types will contribute a result of approximately (A 5, T 225, C 5, G 5, O 10). The summation error mode distribution of these results will approximate (A 680, T 240, C 20, G 20, O 40).

Comparing this summation error mode distribution result with the scenario lacking polymorphisms (approximately A 900, T 20, C 20, G 20, O 40), it's evident that both "A" and "T" polymorphisms coexist in the sample. The estimated ratio of these polymorphism types is approximately 73.9% (680/(680+240))for "A" and 26.1% (240/(680+240)) for "T", aligning closely with the assumed ratio of 75% to 25%.

As mentioned previously, it's important to note that the error modes and their probability profiles may vary across different single molecule sequencing platforms. However, the methodology outlined above remains applicable across all platforms.

The sensitivity and accuracy of polymorphism detection increase with the quantity of original sequence reads collected within a cluster. For instance, with a cluster of 1000 original reads, polymorphisms present at levels as low as 5% can be detected. With a larger cluster of 10,000 original reads, the sensitivity of polymorphism detection increases to approximately 0.5%.

This methodology is capable of detecting various types of polymorphisms, including insertions, deletions, and substitutions. Moreover, it can identify multiple polymorphic positions within a single assay.

The testing fragments subjected to sequencing using this method are those initially captured by the capturing primer, indicating sequence complementarity. Subsequently, these fragments must hybridize successfully with the sequencing primer, and the resultant sequencing reads must attain a predetermined similarity threshold with the reference sequence. This process enables confident target detection within the test sample. Sequence disparities between the sequencing targets and the reference sequence indicate polymorphism presence. Moreover, the ratio of polymorphisms detected in the sequencing reads accurately reflects the polymorphism ratio present in the testing samples.

The template molecules are generated only after hybridization between the capture primers and the complementary target nucleic acid molecules in the sample, and the original sequences are generated only after obtaining the template molecules, such that the number of original sequences in the cluster should be proportional to the number of target nucleic acid molecules in the sample. When the target nucleic acid molecule detected is mRNA, the number of original sequences should be proportional to the expression level of the gene. In other words, the method of this embodiment can simultaneously detect number, concentrations and expression level of multiple genes.

Utilizing this methodology, one can effectively measure the copy number of specific genes present within a testing sample. For example, sample A and sample B are both detected same target fragment by the method, with same sample amount, procedures and same single molecule sequencing conditions. The sample A is collected 100 qualified original sequence reads in the cluster; and sample B collected 500 qualified original sequence. Then we can say sample B contains more target DNA molecules than sample A.

Additionally, gene expression profiling is attainable using RNA samples as sequencing materials. For example, RNAs of gene A and Gene B are both tested in a sample by this method. The collected cluster of gene A having 2000 qualified original sequence; gene B having 30 qualified original sequence. Then it can calculate the relative expression level of gene A and gene B by number of qualified original sequence, in this case, 2000 to 30. This approach simplifies and expedites the complex and time-consuming process of single molecule sequencing technology, thereby broadening its applicability in understanding and addressing biological processes and disease mechanisms.

The method for detecting nucleic acid molecule in this embodiment may detect whether there is a first target nucleic acid molecule in a sample, number of the first target nucleic acid molecule, whether there is a variation in the first target nucleic acid molecule, and ratio and type of the variation in the first target nucleic acid molecule, but the invention is not limited thereto. For example, the number of the first original sequences in the first cluster may be used to estimate the number of the first target nucleic acid molecules in the sample.

In some embodiments, when the first target nucleic acid molecule is DNA with methylation modification, a part of the first target nucleic acid molecule may be directly sequenced, and another part of the first target nucleic acid molecule may be first converted by bisulfite and then sequenced. In some embodiments, when the first target nucleic acid molecule is DNA with methylation modification, a part of the first target nucleic acid molecule and another part of the first target nucleic acid molecule that have been converted by bisulfite may be mixed first and then sequenced.

In some embodiments, the sample may further include a plurality of second target nucleic acid molecules having nucleic acid sequences different from the first target nucleic acid molecules, and the method may further include the following steps: hybridizing a plurality of second capture primers to the plurality of second target nucleic acid molecules to form a plurality of second capture primer-second target nucleic acid molecule complexes; performing a nascent strand synthesis reaction to form a plurality of second template molecule-second target nucleic acid molecule complexes; dissociating a plurality of second template molecules; immobilizing a plurality of second template molecules on a sequencing chip; hybridizing a plurality of second sequencing primers to the plurality of dissociated second template molecules, and sequencing the plurality of second template molecules by single-molecule sequencing to obtain a plurality of second original sequences; comparing the plurality of second original sequences and a second target sequence to calculate a second sequence similarity of each of the plurality of second original sequences; collecting a plurality of second original sequences whose second sequence similarity reaches a predetermined threshold to obtain a second cluster; and analyzing the second cluster by bioinformatics to obtain a second consensus sequence. That is, in some embodiments, the method may be used to simultaneously detect the first target nucleic acid molecule and the second target nucleic acid molecule in the sample. In some embodiments, the method may also be used to simultaneously detect more than two different target nucleic acid molecules from the same sample so that the method may have the effect of multiplex detection or multiplex assay.

A kit (not shown) for detecting nucleic acid molecule in this embodiment may use the aforementioned method to detect the plurality of first target nucleic acid molecules 110 and a plurality of second target nucleic acid molecules in the sample S simultaneously. The kit may include the mix of the plurality of first capture primers 130 and the plurality of second capture primers, the mix of the plurality of first sequencing primers 150 and the plurality of second sequencing primers, a first reagent (such as the polymerase P1 and the deoxyribonucleoside triphosphate N1, etc.) for performing a nascent strand synthesis reaction, the sequencing chip 200, and a second reagent (such as the polymerase P2 and the fluorescence-labelled nucleotide analogs N2, etc.) for performing single-molecule sequencing. The plurality of first target nucleic acid molecules 110 and the plurality of second target nucleic acid molecules respectively have different nucleic acid sequences. The kit for detecting nucleic acid molecule in this embodiment may simultaneously detect a plurality of types of target nucleic acid molecules (such as the first target nucleic acid molecule 110 and the second target nucleic acid molecule, but the invention is not limited thereto), and has the effect of multiplex detection or multiplex assay.

A cartridge (not shown) for detecting nucleic acid molecule in this embodiment may use the aforementioned method to detect the plurality of first target nucleic acid molecules 110 and a plurality of second target nucleic acid molecules in the sample S simultaneously. The cartridge includes a first chamber for adding the sample S during detection, a second chamber containing the mix of the plurality of first capture primers 130 and the plurality of second capture primers and the sequencing chip 200, and a third chamber containing the mix of the plurality of first sequencing primers 150 and the plurality of second sequencing primers, a fourth chamber containing a first reagent (such as the polymerase P1 and the deoxyribonucleoside triphosphate N1, etc.) for performing the nascent strand synthesis reaction, and a fifth chamber containing a second reagent (such as the polymerase P2 and the fluorescence-labelled nucleotide analogs N2, etc.) for performing single-molecule sequencing, and the plurality of first capture primers130 and the plurality of second capture primers are immobilized on the sequencing chip 200. The plurality of first target nucleic acid molecules 110 and the plurality of second target nucleic acid molecules respectively have different nucleic acid sequences. The cartridge for detecting nucleic acid molecule in this embodiment may simultaneously detect a plurality of types of target nucleic acid molecules (such as the first target nucleic acid molecule 110 and the second target nucleic acid molecule, but the invention is not limited thereto), and has the effect of multiplex detection or multiplex assay.

In some embodiments of cartridge for detecting nucleic acid molecule, a plurality of first capture primers and a plurality of first sequencing primers may not be added to a second chamber and a third chamber respectively in advance; on the contrary, after first target nucleic acid molecule to be detected is confirmed, a plurality of required first capture primers and a plurality of required first sequencing primers are added to a second chamber and a third chamber respectively during detection (customized panel assay).

Other embodiments will be provided and described below. It is noted herein that the reference numerals and part of the descriptions of the above embodiment apply to the following embodiments, where the same numerals are used to represent the same or similar components, and descriptions of the same technical contents are omitted. Reference may be made to the above embodiment for the descriptions of the omitted contents, which will not be repeated in the following embodiments.

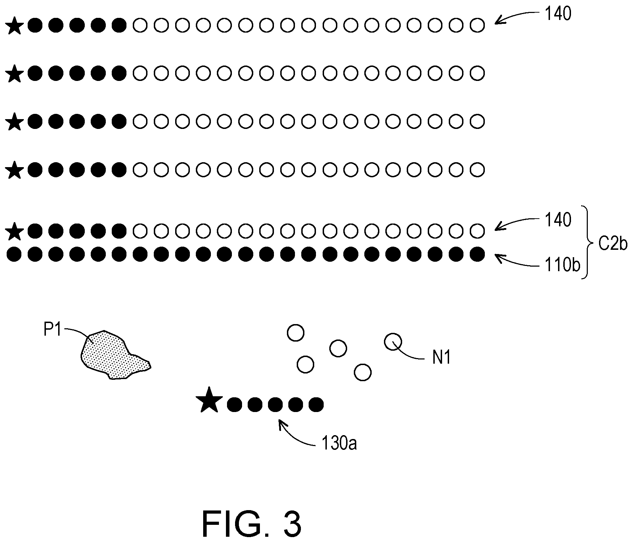

FIG. 3 is a schematic diagram of a method for detecting nucleic acid molecules according to another embodiment of the present invention. FIG. 3 shows a step continuing FIG. 2B and replacing FIG. 2C. The same materials or methods may apply to the same or similar components in the embodiment of FIG. 3 and the embodiment of FIG. 2A to FIG. 2I, so the same and similar descriptions in the two embodiments will not be repeated herein, and the main difference between the two embodiments will be described.

Specifically, referring to FIG. 3, according to steps similar to FIG. 2C, a nascent strand synthesis reaction is performed to form a first template molecule-first target nucleic acid molecule complex C2b. However, since the quantity of the first target nucleic acid molecule used in this embodiment may not be sufficient for detection, a linear amplification is performed after the first template molecule-first target nucleic acid molecule complex C2b is formed; thereby, number of the first template molecules may be increased and detection sensitivity may be improved.

In this embodiment, the linear amplification uses asymmetric polymerase chain reaction (asymmetric PCR) to perform several cycles of the nascent strand synthesis reaction. Each cycle may include the following steps: first using heating to dissociate the first template molecule 140 and the first target nucleic acid molecule 110b in the first template molecule-first target nucleic acid molecule complex C2b, and then annealing the unreacted first capture primer 130 to the dissociated first target nucleic acid molecule 110b, and then perform the nascent strand synthesis reaction using a polymerase P1 and deoxyribonucleoside triphosphates (dNTP) N1.

Compared to general detection methods that use template molecules which are exponentially amplified by a conventional polymerase chain reaction (conventional PCR) for detection, this embodiment uses the first template molecule which are linearly amplified by the asymmetric PCR to linear amplify for detection, thereby preventing the sequence of the detected first template molecule from being distorted by exponential amplification. For example, sequence of the template molecules exponentially amplified by the conventional polymerase chain reaction usually has a certain probability of errors (such as mutations), and the errors (mutations) may also be exponentially amplified through continued conventional polymerase chain reactions, resulting in distortion of the sequence of the exponentially amplified template molecules.

Hereinafter, the method for detecting the nucleic acid molecule in the above embodiment is described in detail by experimental examples. However, the following experimental examples below are not intended to limit the invention.

### [Experimental Example 1] Virus detection

Mutations in S gene of virus SARS-CoV-2 may result in the emergence and continued transmission of multiple variants of concern (VOC). Therefore, it is urgent to develop methods that may quickly detect new virus variants.

In this experimental example, a capture primer containing biotin (Biotin-S-VOC-F) and a sequencing primer (S-VOC-R) were first designed. The nucleic acid sequence of the capture primer containing biotin (Biotin-S-VOC-F) is 5'-TTGGAACAGGAAGAGAATCAGC-3' (SEQ ID NO: 1), and the nucleic acid sequence of the sequencing primer (S-VOC-R) is 5'-TGACACCACCAAAAGAACATGG-3' (SEQ ID NO: 2). The capture primer containing biotin (Biotin-S-VOC-F) and the sequencing primer (S-VOC-R) may be used to detect the nucleic acid sequence of the S gene that encoding the amino acid at position 360 to the amino acid at position 588 of the S protein. The amino acid at position 360 to the amino acid at position 588 of the S protein are located in a receptor-binding domain (RBD) where most important mutations in the S protein occur.

Then, after extracting RNA from the virus collected from positive nasopharyngeal swab samples, the RNA in the virus is detected according to the flow chart of FIG. 1 and the schematic diagrams of FIG. 2A to FIG. 2I to quickly identify whether the virus is a known virus strain or a new virus variant.

### [Experimental Example 2] Bacteria detection

Treatment of sepsis often requires to identify pathogenic bacteria in the blood so that right antibiotics may be used to kill the pathogenic bacteria. Currently, clinical methods used to identify the pathogenic bacteria in blood are mainly bacterial culture and molecular diagnosis (such as PCR). However, bacterial culture is time-consuming (at least 24 hours to 72 hours), and some bacteria are not easy to culture. Molecular diagnosis using PCR often results in false positive results (for example, unexpected nucleic acid molecules are amplified) or false negative results (for example, mutation occurs in the nucleic acid molecule that should be annealed by the primer).

In this experimental example, a capture primer containing biotin (16S-F) and a sequencing primer (16S-R) were first designed. The nucleic acid sequence of the capture primer containing biotin (16S-F) is 5'-CAGACTCCTACGGGGAGGCAGCAGT-3' (SEQ ID NO: 3), and the nucleic acid sequence of the sequencing primer (16S-R) is 5'-ACTTAACCCAACATCTCACGACAC-3' (SEQ ID NO: 4). The capture primer containing biotin (16S-F) and the sequencing primer (16S-R) may be used to detect a nucleic acid molecule with 762 nucleotides in conserved region of bacterial 16S rRNA gene.

Then, after DNA is extracted from the bacteria collected from patient's blood, the DNA is detected according to the flow chart of FIG. 1 and the schematic diagrams of FIG. 2A to FIG. 2I to quickly identify the type of the pathogenic bacteria.

### [Experimental Example 3] Human leukocyte antigen gene detection

The major histocompatibility complex (MHC) encoded by the human leukocyte antigen (HLA) gene is an important regulator of immune responses and drug hypersensitivity reactions. In recent years, genotyping of human leukocyte antigen genes has been used to understand the antigen presentation and immune recognition of the major histocompatibility complex, which may be used to promote development of personalized immunotherapy, thereby improving patient outcomes. Usually, in pairs of chromosomes, the allele type of the HLA locus on one chromosome is different from the allele type of the HLA locus on the other chromosome, and the sequence difference between the two different allele types is less than 4 %. The HLA genes in each allele type may include multiple different genotyping, such as HLA-A, HLA-B, HLA-C, HLA-DRB1, HLA-DQB1, HLA-DPB1, HLA- DQA1 etc.

In this experimental example, a capture primer containing biotin (HLA-A_F-267) and a sequencing primer (HLA-A_R-592) were first designed based on the reference sequence of the HLA-A gene (GenBank: OQ104609.1). The nucleic acid sequence of the capture primer containing biotin (HLA-A F-267) is 5'-CCACCGGGACTCAGATTCTC-3' (SEQ ID NO: 5), and the nucleic acid sequence of the sequencing primer (HLA-A_R-592) is 5'-TGTCGTCCACGTAGCCCAC-3' (SEQ ID NO: 6). The capture primer containing biotin (HLA-A_F-267) and the sequencing primer (HLA-A_R-592) may be used to detect a nucleic acid molecule having 325 nucleotides in the conserved region of HLA-A gene that does not have single nucleotide polymorphism (SNP).

Then, after the genomic DNA is extracted, an asymmetric polymerase chain reaction is used to amplify the number of template molecules. Specifically, the asymmetric polymerase chain reaction was performed in 50 µl of reaction solution using 1 µg of genomic DNA, 0.5 µM capture primer (HLA-A_F-267), and 0.005 µM amplify primer. The conditions for the asymmetric polymerase chain reaction are:

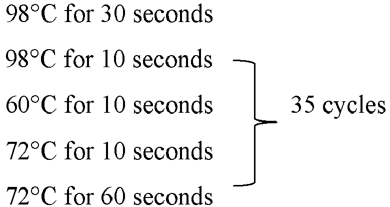

The reaction time of the asymmetric polymerase chain reaction is approximately 33 minutes to 42 minutes.

Then, after the asymmetric polymerase chain reaction is performed, a spin column is used to remove the unreacted capture primer (HLA-A_F-267) and purify the amplified template molecule.

Then, the HLA-A gene is detected according to the flow chart of FIG. 1 and the schematic diagrams of FIG. 2A to FIG. 2I. Specifically, the method for performing single-molecule sequencing in this experimental example includes the following steps: applying 50 µl of 10 nM streptavidin on a coverslip, and reacting for 30 minutes to obtain a streptavidin-coated coverslip. Next, the streptavidin-coated coverslip is washed three times with a buffer (including 50mM Tris-HCl (pH7.5), 10mM MgOAc, and 1mM DTT). Next, 20ul of 100nM template molecules "biotin-HLAA-325" were immobilized on the streptavidin-coated coverslip. Then, the coverslip immobilized with the template molecules is placed on the heating plate, 20ul of 100nM sequencing primer (HLA-A_R-592) is added to anneal the template molecules on the coverslip. After reaction for 5 minutes, a polymerase and fluorescence-labelled nucleotide analogs are added to initiate sequencing reaction. Then, an optical imaging system (including total internal reflection fluorescence microscopy (TIRFM), laser, filter, and image recorder) is used to record the fluorescence signal in the sequencing reaction in real time. The sequencing reaction takes about 30 minutes.

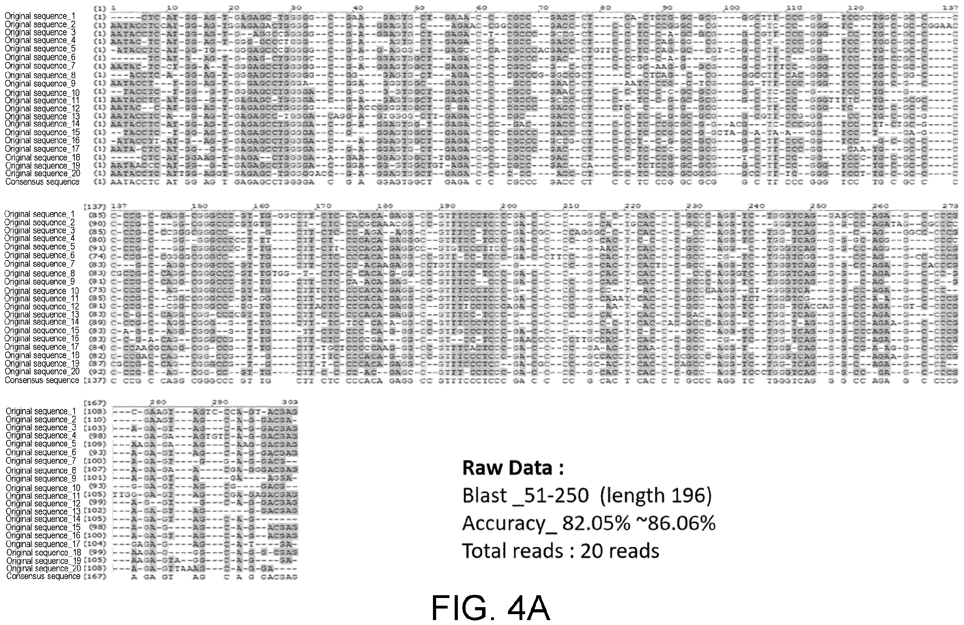

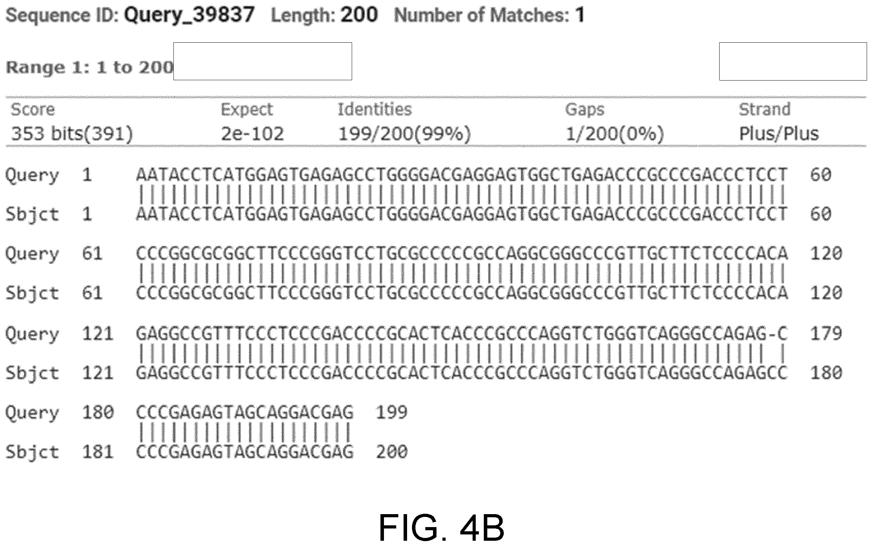

In this experimental example, the method for obtaining the consensus sequence includes the following steps: using a base calling algorithm to analyze the fluorescent signal and convert the fluorescent signal into the corresponding nucleotide to obtain 4219 original sequences. Next, the ClustalW tool of Bioedit software is used to compare the 4219 original sequences with the HLA-A reference sequence to calculate the sequence similarity of the 4219 original sequences. Next, 20 original sequences with a sequence similarity greater than 82% and a sequence length greater than 80% of the HLA-A reference sequence are collected from the 4219 original sequences to obtain a cluster. Next, 20 original sequences in the cluster are analyzed by bioinformatics to obtain a consensus sequence. As shown in FIG. 4A, the consensus sequence is generated by aligning and analyzing 20 original sequences with the sequence similarity ranging from 82.05% to 86.06%. Next, as shown in FIG. 4B, the consensus sequence is compared with the HLA-A reference sequence, and sequence similarity or accuracy of the consensus sequence is calculated. The result shows that the sequence similarity or accuracy of the consensus sequence may be improved to approximately 99.5%.

In summary, in the method, the kit and the cartridge for detecting nucleic acid molecule according to an embodiment of the present invention, the detection steps are simplified, the detection time is reduced and the detection accuracy is improved by using a specific sequence of steps. The specific sequence of steps at least includes the following steps: first hybridizing the capture primers to the target nucleic acid molecules in the unpurified or crudely purified sample, then obtaining the template molecules by the nascent strand synthesis reaction, then hybridizing the sequencing primers to the template molecules, then obtaining original sequences of the template molecules by the single-molecule sequencing, and then analyzing the original sequences whose sequence similarity reaches a predetermined threshold to obtain the consensus sequence. In this embodiment, the specific sequence of steps combines a simple preparation method and bioinformatics. The simple preparation method targets nucleic acid fragments read during the single-molecule sequencing and reads the nucleic acid fragments across multiple genomes in the sample. The bioinformatics assembles the low-accuracy original sequences obtained from each individually read nucleic acid fragment into a high-accuracy result that can meet application requirements.

## Claims

1. A method for detecting nucleic acid molecule, comprising:
providing a sample (S), wherein the sample (S) comprises a plurality of first target nucleic acid molecules (110, 110b);
hybridizing a plurality of first capture primers (130) to the plurality of first target nucleic acid molecules (110, 1 10b) to form a plurality of first capture primer-first target nucleic acid molecule complexes (C1);
performing a nascent strand synthesis reaction to form a plurality of first template molecule-first target nucleic acid molecule complexes (C2, C2b);
dissociating a plurality of first template molecules (140);
hybridizing a plurality of first sequencing primers (150) to the plurality of dissociated first template molecules (140), and sequencing the plurality of first template molecules (140) by single-molecule sequencing to obtain a plurality of first original sequences (160, 161, 162, 163);
comparing the plurality of first original sequences (160, 161, 162, 163) and a first target sequence to calculate a first sequence similarity of each of the plurality of first original sequences (160, 161, 162, 163);
collecting a plurality of first original sequences (163) whose first sequence similarity reaches a predetermined threshold to obtain a first cluster (170); and
analyzing the first cluster (170) by bioinformatics to obtain a first consensus sequence (180),
wherein the plurality of first capture primers (130) have linkers (132), and the linkers (132) are paired with another linker (210) on a sequencing chip (200).

2. The method according to claim 1, wherein the sample (S) is unpurified or crudely purified and further comprises a plurality of non-target nucleic acid molecules (120), and the plurality of non-target nucleic acid molecules (120) do not hybridize by the plurality of first capture primers (130) or the plurality of first sequencing primers (150).

3. The method according to claim 1, wherein the plurality of first target nucleic acid molecules (110, 110b) are not amplified by polymerase chain reaction.

4. The method according to claim 1, wherein the plurality of first target nucleic acid molecules (110, 110b) comprise DNA, mRNA, miRNA or miniRNA.

5. The method according to claim 1, wherein pairing method comprises biotin-streptavidin binding, nucleic acid hybridization or chemical bonding.

6. The method according to claim 1, wherein the plurality of first template molecules (140) are single-stranded and linear nucleic acids.

7. The method according to claim 1, wherein the plurality of first template molecules (140) have 25 to 10,000 nucleotides.

8. The method according to claim 1, further comprising:
after forming the plurality of first template molecule-first target nucleic acid molecule complexes (C2, C2b), performing a cleaning process and a purification process.

9. The method according to claim 1, further comprising:
after dissociating the plurality of first template molecules (140) and before hybridizing the plurality of first sequencing primers (150) to the plurality of dissociated first template molecules (140), immobilizing the plurality of dissociated first template molecules (140) on a sequencing chip (200).

10. The method according to claim 1, further comprising:
before hybridizing the plurality of first capture primers (130) to the plurality of first target nucleic acid molecules (110, 110b), immobilizing the plurality of first capture primers (130) on a sequencing chip (200).

11. The method according to claim 1, wherein the plurality of first template molecules (140) comprises a first end (141) and a second end (142), the first end (141) comprises the first capture primer (130), and the second end (142) is hybridized by the plurality of first sequencing primers (150).

12. The method according to claim 1, further comprising:
calculating an error mode distribution of each nucleotide in the first consensus sequence (180) to determine whether the plurality of first target nucleic acid molecules (110, 1 10b) have variation.

13. The method according to claim 12, wherein the variation comprises nucleotide insertion, nucleotide deletion, nucleotide substitution, gene recombination and/or methylation site.

14. The method according to claim 1, wherein the predetermined threshold is greater than 80%.

15. The method according to claim 1, wherein the sample (S) further comprises a plurality of second target nucleic acid molecules, and the method further comprises:
hybridizing a plurality of second capture primers to the plurality of second target nucleic acid molecules to form a plurality of second capture primer-second target nucleic acid molecule complexes;
performing a nascent strand synthesis reaction to form a plurality of second template molecule-second target nucleic acid molecule complexes;
dissociating a plurality of second template molecules;
hybridizing a plurality of second sequencing primers to the plurality of dissociated second template molecules, and sequencing the plurality of second template molecules by single-molecule sequencing to obtain a plurality of second original sequences;
comparing the plurality of second original sequences and a second target sequence to calculate a second sequence similarity of each of the plurality of second original sequences;
collecting a plurality of second original sequences whose second sequence similarity reaches a predetermined threshold to obtain a second cluster; and
analyzing the second cluster by bioinformatics to obtain a second consensus sequence.

16. The method according to claim 15, further comprising:
simultaneously detecting the plurality of first target nucleic acid molecules (110, 110b) and the plurality of second target nucleic acid molecules.

17. The method according to claim 1, wherein the method counts the number of the plurality of first original sequences (160, 161, 162, 163) in the cluster to determine copy number and expression level of the plurality of first target nucleic acid molecules (110, 1 10b).

18. A kit for detecting nucleic acid molecule, wherein the kit uses the method according to claim 1 to detect a plurality of first target nucleic acid molecules (110, 110b) and a plurality of second target nucleic acid molecules in a sample (S) simultaneously, the kit comprises a mix of a plurality of first capture primers (130) and a plurality of second capture primers, a mix of a plurality of first sequencing primers (150) and a plurality of second sequencing primers, a first reagent for performing a nascent strand synthesis reaction, a sequencing chip (200), and a second reagent for single-molecule sequencing.

19. The kit according to claim 18, wherein the capture primers and the sequencing primers are not provided in advance; on the contrary, after the plurality of first target nucleic acid molecules (110, 1 10b) to be detected is confirmed, a plurality of required capture primers and a plurality of required sequencing primers are added to the kit.

20. A cartridge for detecting nucleic acid molecule, wherein the cartridge uses the method according to claim 1 to detect a plurality of first target nucleic acid molecule and a plurality of second target nucleic acid molecules in a sample (S) simultaneously, the cartridge comprises a first chamber for adding the sample (S) during detection, a second chamber containing a mix of a plurality of first capture primers (130) and a plurality of second capture primers and a sequencing chip (200), and a third chamber containing a mix of plurality of first sequencing primers (150) and a plurality of second sequencing primers, a fourth chamber containing a first reagent for performing a nascent strand synthesis reaction, and a fifth chamber containing a second reagent for single-molecule sequencing, and the plurality of first capture primers (130) and the plurality of second capture primers are immobilized on the sequencing chip (200).

21. The cartridge according to claim 20, the capture primers and the sequencing primers are not provided in advance; on the contrary, after the plurality of first target nucleic acid molecules (110, 1 10b) to be detected is confirmed, a plurality of required capture primers and a plurality of required sequencing primers are added to the chambers of cartridge.
